# EUROPEAN PATENT APPLICATION

(11) **EP 3 733 831 A1**
(43) Date of publication of application: **04.11.2020**
(21) Application number: 18895199.0
(22) Date of filing: 28.12.2018
(51) Int. Cl.: C12M 1/00

(54) **ARTIFICIAL TISSUE PERFUSION DEVICE AND METHOD OF DRUG ASSESSMENT USING ARTIFICIAL TISSUE**

(30) Priority: 28.12.2017 US 201762611338 P
(71) Applicant: The University of Tokyo, Bunkyo-ku, Tokyo 113-8654 (JP)
(72) Inventor: TAKEUCHI Shoji, Tokyo 113-8654 (JP); MORIMOTO Yuya, Tokyo 113-8654 (JP); NAGATA Shogo, Tokyo 113-8654 (JP)
(74) Representative: Ernest Gutmann - Yves Plasseraud S.A.S.
(86) International application number: PCT/JP2018/048493
(87) International publication number: WO 2019/132008

(57) **Abstract**

An object of the present invention is to provide an artificial tissue perfusion device capable of analyzing the interaction between a vascular cell layer and a parenchymal cell layer with high accuracy. An artificial tissue perfusion device includes a co-culture system (C) in which a plurality of types of cell are cultured. The co-culture system has a tubular well part (10) having a culture space (11) inside; a base material (20) having a perfusion flow path (26) which extends in a predetermined direction and is perfused with a medium, and a holding part (23) which opens to the perfusion flow path and holds the well part attachably and detachably; and a gel membrane (30) having a form of a porous membrane and disposed at an end portion of the well part facing the perfusion flow path in a case where the well part is held by the holding part. A tissue-based cell is cultured on a surface side of the gel membrane facing the culture space, and a luminal cell is cultured on a surface side of the gel membrane facing the perfusion flow path.

## Description

### [Technical Field]

The present invention relates to an artificial tissue perfusion device and a method of drug assessment using an artificial tissue.

Priority is claimed on United States Provisional Application No. 62/611,338, filed December 28, 2017, the content of which is incorporated herein by reference.

### [Background Art]

A technique using Transwell (registered trademark) having a porous membrane is known as, for example, a system for assessing the interaction between a vascular cell layer and a parenchymal cell layer in a state where flow stimulation by a blood flow is applied (for example, Non-Patent Document 1).

### [Citation List]

### [Non-Patent Literature]

[Non-Patent Document 1]
Lab Chip 2013, 13, 3538-3547

### [Summary of Invention]

### [Technical Problem]

However, cell polarity depending on the blood flow could not be controlled with the above technique, and it was difficult to perform precise analysis.

The present invention has been made in view of the above-mentioned problem, and an object thereof is to provide an artificial tissue perfusion device capable of analyzing the interaction between a vascular cell layer and a parenchymal cell layer with high accuracy, and a method of drug assessment using an artificial tissue.

### [Solution to Problem]

According to a first aspect of the present invention, there is provided an artificial tissue perfusion device including a co-culture system in which a plurality of types of cell are cultured, in which the co-culture system has a tubular well part having a culture space inside, a base material having a perfusion flow path which extends in a predetermined direction and is perfused with a medium, and a holding part which opens to the perfusion flow path and holds the well part attachably and detachably, and a gel membrane having a form of a porous membrane and disposed at an end portion of the well part facing the perfusion flow path in a case where the well part is held by the holding part, and in which a tissue-based cell is cultured on a first surface side of the gel membrane facing the culture space, and a luminal cell is cultured on a second surface side of the gel membrane facing the perfusion flow path.

According to a second aspect of the present invention, there is provided a method of drug assessment including manufacturing an artificial tissue using the artificial tissue perfusion device according to the first aspect of the present invention; bringing a drug into contact with the artificial tissue; and measuring a response of the artificial tissue to a stimulus caused by the contact with the drug or permeability of the drug into the artificial tissue.

### [Advantageous Effects of Invention]

According to the present invention, it is possible to provide an artificial tissue perfusion device capable of analyzing the interaction between a vascular cell layer and a parenchymal cell layer with high accuracy, and a method of drug assessment capable of assessing the interaction between a vascular cell layer and a parenchymal cell layer with respect to a drug with high accuracy.

### [Brief Description of Drawings]

Fig. 1 is a schematic configuration diagram of a perfusion system SYS including a perfusion device 1 according to the present invention.
Fig. 2 is a plan view of the perfusion device 1 viewed from a well part 10 side.
Fig. 3 is a cross-sectional view in which an inner peripheral surface of a cap member 40 is fitted to an outer peripheral surface of the well part 10.
Fig. 4 shows graphs showing the relationship between flow stimulation and gene expression of neural stem cells (upper part) and vascular endothelial cells (lower part).
Fig. 5 is a table showing measurement results of the amount of albumin produced for each of gel membranes.
Fig. 6A is a graph showing the relationship between flow stimulation and the amount of albumin produced, Fig. 6B is a graph showing the relationship between flow stimulation and an amount of albumin permeated, and Fig. 6C is a graph showing the relationship between flow stimulation and barrier function.
Fig. 7 is a diagram showing fluorescence images of vascular endothelial cells responding to flow stimulation.
Fig. 8 is a cross-sectional view showing a modification example of a pre-culture part.
Fig. 9 is a cross-sectional view showing a modification example of a medium reservoir part.
Fig. 10 is a cross-sectional view showing a modification example of the medium reservoir part.

### [Description of Embodiments]

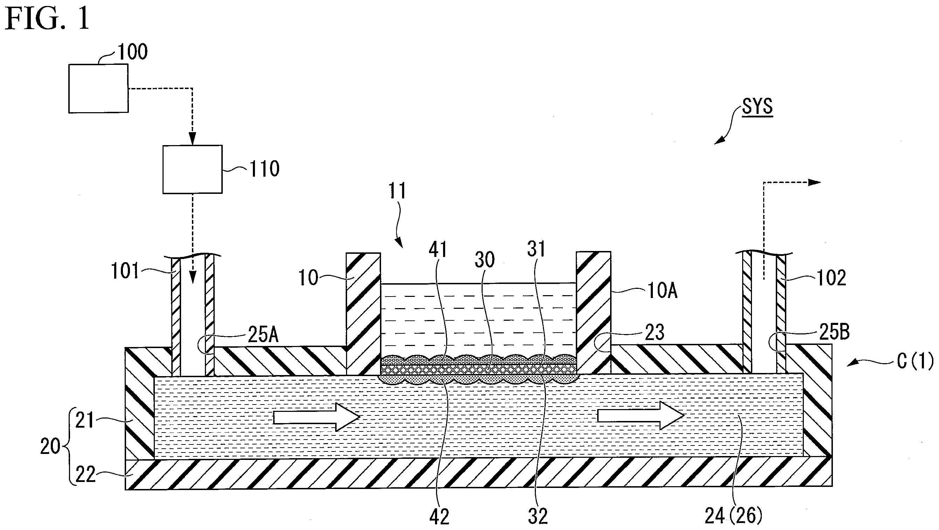

Hereinafter, embodiments of an artificial tissue perfusion device and a method of drug assessment using the artificial tissue perfusion device of the present invention will be described with reference to Figs. 1 to 8. The following embodiments show one aspect of the present invention and therefore do not limit the present invention. The embodiments can be arbitrarily modified within the scope of the technical idea of the present invention. In addition, in the following figures, the scale, number, and the like in each structure may be different from those of the actual structure to make each configuration easy to understand.

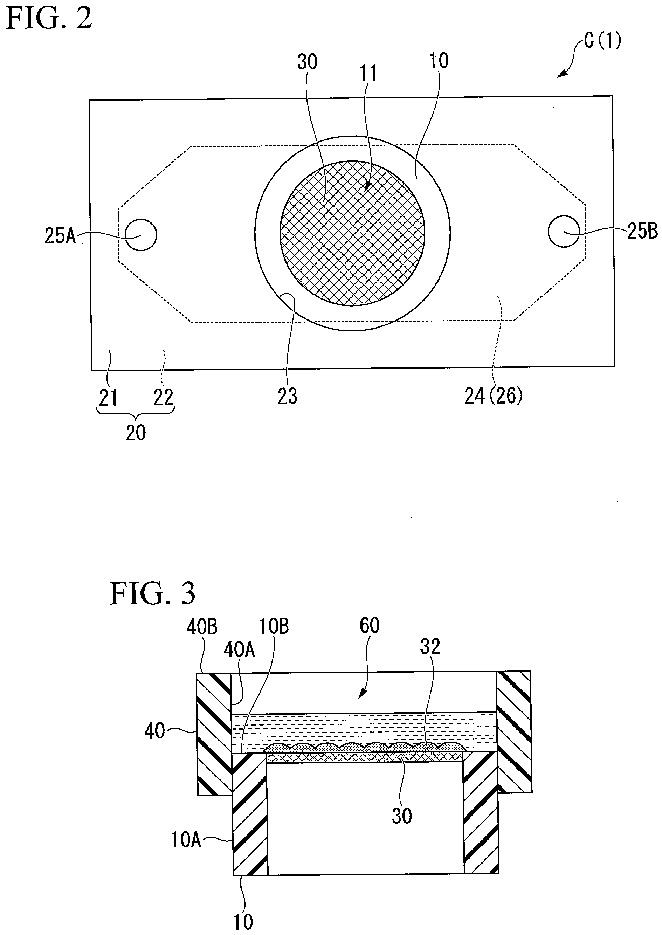

Fig. 1 is a schematic configuration diagram of a perfusion system SYS including a perfusion device (artificial tissue perfusion device) 1 according to the present invention. Fig. 2 is a plan view of the perfusion device 1 viewed from a well part 10 side.

The perfusion system SYS includes a perfusion device 1, a medium supply part 100, a supply pipe 101, a discharge pipe 102, and an adjustment part 110.

The medium supply part 100 stores a medium and supplies the medium to the perfusion device 1. One end of the supply pipe 101 is connected to the perfusion device 1. The supply pipe 101 introduces the medium supplied from the medium supply part 100 into the perfusion device 1. The discharge pipe 102 discharges the medium from the perfusion device 1.

The adjustment part 110 adjusts an introduction amount of the medium supplied from the medium supply part 100 and introduced into the perfusion device 1, and thereby adjusts a flow rate of the medium in the perfusion device 1. The adjustment part 110 is, for example, a pump provided in the middle of the supply pipe 101. A flow rate of the medium in the perfusion device 1 can be controlled by adjusting a drive amount of the pump as the adjustment part 110 and adjusting an introduction amount of the medium introduced into the perfusion device 1. The adjustment part 110 may be configured to adjust the amount of the medium supplied from the medium supply part 100.

The perfusion device 1 includes a co-culture system C in which a plurality of types of cell are cultured. The co-culture system C includes a well part 10, a base material 20, a gel membrane 30, and a cap member (pre-culture part) 40 (refer to Fig. 3).

The well part 10 is formed in a tubular shape. For example, the well part 10 is formed of a transparent material and has a cylindrical shape. The well part has a culture space 11 inside. The material of the well part 10 is not particularly limited, and it is possible to use, for example, polystyrene (PS), polycarbonate (PC), polyethylene terephthalate (PET), polytetrafluoroethylene (PTFE), and the like.

The base material 20 has an upper base material 21 and a lower base material 22. As shown in Fig. 2, the upper base material 21 and the lower base material 22 are both formed in a rectangular shape in plan view. The upper base material 21 and the lower base material 22 are joined to each other in a vertical direction by an adhesive or the like, for example. The material of the upper base material 21 is not particularly limited, and examples thereof include elastomers such as silicone rubber and PDMS (polydimethylsiloxane), and the like. The material of the lower base material 22 is not particularly limited, and examples thereof include elastomers such as silicone rubber and PDMS (polydimethylsiloxane), glass, and the like.

The lower base material 22 is formed in a flat plate shape of a rectangular shape in plan view.

The upper base material 21 has a groove part 24 that is open to a surface (lower surface) facing the lower base material 22. The groove part 24 extends in a longitudinal direction (a horizontal direction in Figs. 1 and 2). The groove part 24 is a recess surrounded by a lower surface of the upper base material 21. Distal ends of both end portions of the groove part 24 in the longitudinal direction are formed in a tapered shape. The distal end of the groove part 24 is not limited to a tapered shape, and the groove part 24 may be configured to extend while having a uniform width.

The upper base material 21 has a holding part 23 that is located at the center in the longitudinal direction and the center in a lateral direction and has a circular shape in plan view. The holding part 23 penetrates the upper base material 21 in a thickness direction (a vertical direction in Fig. 1). The holding part 23 is fitted to an outer peripheral surface of the well part 10 in a liquid-tight manner to hold the well part 10 attachably and detachably with respect to the upper base material 21. A lower end surface of the well part 10 faces, for example, a bottom surface of the groove part 24 when the well part 10 is held by the holding part 23.

The upper base material 21 has connection holes 25A and 25B on both outer sides in the longitudinal direction at the center in the lateral direction. The connection holes 25A and 25B respectively penetrate the upper base material 21 in the thickness direction. Lower ends of the connection holes 25A and 25B are respectively open to the groove part 24. The supply pipe 101 is connected to the connection hole 25A. The discharge pipe 102 is connected to the connection hole 25B.

In a case where the upper base material 21 and the lower base material 22 are joined to each other in the vertical direction, a perfusion flow path 26 that is surrounded by the groove part 24 and an upper surface of the lower base material 22 and extends in the longitudinal direction is formed. The supply pipe 101 is connected to one end side of the perfusion flow path 26 in the longitudinal direction, and the discharge pipe 102 is connected to the other end side thereof.

The gel membrane 30 is disposed on the end portion side of the well part 10 facing the perfusion flow path 26 in a case where the well part 10 is held by the holding part 23. The gel membrane 30 has a first surface 31 facing the culture space 11 of the well part 10 and a second surface 32 facing the perfusion flow path 26. In a case where the well part 10 is held by the holding part 23, the second surface 32 faces, for example, the lower end surface of the well part 10 and the bottom surface of the groove part 24.

The gel membrane 30 is formed in a porous membrane shape. The gel membrane 30 contains a vitrified extracellular matrix component. The gel membrane 30 is produced by, for example, drying hydrogel, vitrifying it, and then rehydrating it. The gel membrane 30 is, for example, a vitrified collagen gel membrane, and is formed in a porous membrane shape in which collagen fibers are disposed at a high density.

Tissue-based cells are seeded and then cultured on the first surface 31 of the gel membrane 30 to form a tissue layer 41 (details to be described later). Luminal cells are seeded and then cultured on the second surface 32 of the gel membrane 30 to form a planar luminal layer 42 (details to be described later). The width of the perfusion flow path 26 is preferably set larger than the maximum diameter of the gel membrane 30 such that flow stimulation from a medium perfusing the perfusion flow path 26 is applied to the luminal cells seeded on the second surface 32 of the gel membrane 30. In addition, the thickness (depth) of the perfusion flow path 26 is preferably 1 mm or less to reduce the amount of medium used for the luminal cells and to create a laminar medium flow.

Fig. 3 is a cross-sectional view in which an inner peripheral surface of the cap member 40 is fitted to an outer peripheral surface of the well part 10.

The cap member 40 is used in a case where pre-culture is performed on the second surface 32 before performing co-culture on the first surface 31 and the second surface 32 of the gel membrane 30 by allowing the holding part 23 of the upper base material 21 to hold the well part 10.

For example, the cap member 40 is formed of the same material as those of the upper base material 21 and the lower base material 22 and has a cylindrical shape. An inner peripheral surface 40A of the cap member 40 is fitted to at least an upper side of an outer peripheral surface 10A of the well part 10 in which the gel membrane 30 faces upward. In a case where the well part 10 and the cap member 40 are fitted to each other, an upper end surface 40B of the cap member 40 is provided above an end surface 10B of the well part 10 on the side on which the gel membrane 30 is provided. In a case where the well part 10 and the cap member 40 are fitted to each other, a pre-culture space 60 surrounded by the inner peripheral surface 40A of the cap member 40, the end surface 10B of the well part 10, and the second surface 32 of the gel membrane 30 is formed.

In the pre-culture space 60 formed by fitting the well part 10 and the cap member 40 to each other, luminal cells (details to be described later) are seeded on the second surface 32 of the gel membrane 30, and a planar luminal layer can be pre-cultured on the second surface 32 of the gel membrane 30 by supplying a medium.

In the well part 10 in which the luminal layer is pre-cultured on the second surface 32 of the gel membrane 30, the outer peripheral surface 10A of the well part 10 is fitted to the holding part 23 of the upper base material 21 after separating the cap member 40. Accordingly, the luminal layer pre-cultured on the second surface 32 of the gel membrane 30 is disposed to face the groove part 24 (perfusion flow path 26).

The tissue layer 41 formed of the tissue-based cells formed on the first surface 31 of the gel membrane 30 is not particularly limited. For the tissue layer 41, neural tissue formed of neural cells, muscle tissue formed of skeletal muscle cells or cardiomyocytes, liver tissue formed of hepatocytes, pancreatic tissue formed of pancreatic cells, dermal tissue formed of dermal cells, and the like are exemplary examples. The tissue layer is not limited to a single layer, and a plurality of tissue layers may be laminated. In addition, the tissue layer may be a three-dimensional tissue layer in which a plurality of cells are mixed.

It is possible to select various kinds of media and extracellular matrix components for culturing tissue-based cells.

Examples of the luminal layer 42 formed of the luminal cells formed on the second surface 32 of the gel membrane 30 include a vascular layer formed of vascular cells, a renal tubular layer formed of renal tubular cells, and the like. Vascular cells are preferably vascular endothelial cells. Examples of vascular endothelial cells include cells derived from mammals such as humans, mice, and rats, of which vascular endothelial cells derived from humans are preferable. Examples of vascular endothelial cells derived from humans include human umbilical vein endothelial cells (HUVEC), human umbilical artery endothelial cells (HUAEC), human coronary artery endothelial cells (HCAEC), human saphenous vein endothelial cells (HSaVEC), human pulmonary artery endothelial cells (HPAEC), human aortic endothelial cells (HAoEC), human dermal microvascular endothelial cells (HDMEC), human dermal blood endothelial cells (HDBEC), human dermal lymphatic endothelial cells (HDLEC), human pulmonary microvascular endothelial cells (HPMEC), human cardiac microvascular endothelial cells (HCMEC), human bladder microvascular endothelial cells (HBdMEC), human uterine microvascular endothelial cells (HUtMEC), human brain endothelial cells (HBEC), and the like, of which human umbilical vein endothelial cells (HUVEC) are preferable.

In the perfusion system SYS described above, first, the upper base material 21 and the lower base material 22 described above are connected to each other in the vertical direction using an adhesive or the like. By connecting the upper base material 21 and the lower base material 22 to each other, the perfusion flow path 26 extending in the longitudinal direction is formed. Thereafter, the outer peripheral surface 10A of the well part 10 is fitted to and held by the holding part 23 of the upper base material 21 to allow the second surface 32 of the gel membrane 30 to face the perfusion flow path 26, and thereby the perfusion device 1 is manufactured.

In a case where the well part 10 is held by the holding part 23 of the upper base material 21, an adhesive may be applied between the outer peripheral surface 10A and the holding part 23 to fix the well part 10 in a liquid-tight manner. As the adhesive, the above-mentioned PDMS of a photocurable type (UV curable type) or the like can be used.

Thereafter, the supply pipe 101 connected to the medium supply part 100 via the adjustment part 110 is connected to the connection hole 25A, and the discharge pipe 102 is connected to the connection hole 25B, and thereby the perfusion system SYS is manufactured.

Subsequently, a co-culture method using the perfusion system SYS having the perfusion device 1 will be described.

First, as shown in Fig. 3, the inner peripheral surface 40A of the cap member 40 is fitted to the outer peripheral surface 10A of the well part 10 in which the gel membrane 30 faces upward. Next, the second surface 32 of the gel membrane 30 is coated with an extracellular matrix component, luminal cells are seeded thereon, and a medium is introduced into the pre-culture space 60 to perform pre-culture.

In a case where the luminal cells adhere to the second surface 32 of the gel membrane 30 by pre-culture, the cap member 40 is detached from the well part 10. Next, as shown in Fig. 1, the well part 10 from which the cap member 40 has been detached is turned upside down, and the well part 10 is inserted into and held by the holding part 23 of the upper base material 21 with the gel membrane 30 facing downward. Accordingly, the second surface 32 of the gel membrane 30 to which the luminal cells are attached faces the perfusion flow path 26.

Next, tissue-based cells are seeded on the first surface 31 of the gel membrane 30 exposed at a bottom part of the culture space 11 of the well 10, and a medium is introduced into the culture space 11 to culture the tissue-based cells. In addition to the procedure of allowing the holding part 23 to hold the well part 10, and then seeding the tissue-based cells on the first surface 31 of the gel membrane 30, a procedure of seeding the tissue-based cells on the first surface 31 of the gel membrane 30, and then allowing the holding part 23 to hold the well part 10 may be adopted. In addition, the medium that has been supplied from the medium supply part 100 and of which an amount has been adjusted by the adjustment part 110 is introduced into the perfusion flow path 26 from the supply pipe 101. Because a width of the perfusion flow path 26 gradually increases from the end portion toward the center in the longitudinal direction, the medium introduced from the supply pipe 101 into the perfusion flow path 26 is smoothly filled into the perfusion flow path 26 and perfuses therein. The medium perfusing the perfusion flow path 26 is discharged from the discharge pipe 102.

As a result, the tissue-based cells seeded on the first surface 31 of the gel membrane 30 are cultured by the medium introduced into the culture space 11, and the luminal cells attached to the second surface 32 of the gel membrane 30 are cultured in the medium introduced into the perfusion flow path 26 and perfused therein. That is, in the perfusion device 1, the tissue-based cells and the luminal cells are co-cultured in a state where the flow stimulation is applied thereto, and it is possible to reproduce, for example, actual environments around the blood vessel and the like.

In addition, according to the perfusion device 1 of the present embodiment, by adjusting the adjustment part 110, it is also possible to compare and assess a case of co-culture in a state where the perfusion flow path 26 is perfused with a medium and a case of co-culture in a state where the perfusion flow path 26 is filled with a medium without being perfused therewith. Furthermore, regarding the case of co-culture in a state where the perfusion flow path 26 is perfused with a medium, it is also possible to compare and assess cases in which the adjustment part 110 is adjusted to change the amount of a medium introduced into the perfusion flow path 26, and a flow rate of the medium in the perfusion flow path 26 is changed.

Furthermore, in the present embodiment, the perfusion device 1 may include a plurality of perfusion flow paths 26, holding parts 23, and well parts 10 which are respectively partitioned from each other. In this case, by branching the supply pipe 101 into a plurality of parts such that a medium can be introduced into each of the plurality of perfusion flow paths 26, and providing the adjustment part 110 to each branched part of the supply pipe 101, it is also possible to compare and assess at the same time the above-described cases such as the presence or absence of perfusion of a medium in the perfusion flow path 26, and different flow rates of a medium.

### (Assessment method)

As still another aspect, the present invention relates to a method of assessing stimulating properties of contact with a drug in an artificial tissue produced by using the perfusion device 1 according to the present invention. A drug in the present invention includes medicines such as pharmaceutical products, cosmetic products, quasi-pharmaceutical products, and the like. According to the assessment method of the present invention, for example, the drug can be assessed in an environment close to the actual surroundings and the like of the luminal layer as compared with conventional methods. In addition, the assessment method of the present invention is extremely useful, for example, in dynamic assessment of medicines having various molecular weights in creation (screening) of new drugs, and assessment in development of cosmetic products, quasi-pharmaceutical products, and the like.

The assessment method of the present invention can be performed, for example, by bringing a drug into contact with an artificial tissue, and measuring a response of the artificial tissue to a stimulus caused by the contact with the drug or permeability of the drug into the artificial tissue. The response can be measured, for example, by measuring transcutaneous and transendothelial electrical resistance (barrier function). The drug refers to a substance as an assessment target, and examples thereof include an inorganic compound, an organic compound, and the like.

Regarding bringing a drug into contact with an artificial tissue, for example, it is possible to apply a drug to the tissue layer 41 to assess absorption of the drug into the perfusion flow path 26, or it is possible to mix a drug into a medium introduced into the perfusion flow path 26 to assess diffusion/permeation of the drug from the perfusion flow path 26 into the tissue layer 41.

### [Examples]

### [Experimental Example 1]

In the perfusion device 1 of the above embodiment, a vitrigel membrane was used as the gel membrane 30. The vitrigel membrane was produced, in which vascular endothelial cells (HUVEC) were bonded to and cultured on the second surface 32 of the vitrigel membrane on the side of the perfusion flow path 26, and neural stem cells (NSC) were bonded to and cultured on the first surface 31 of the vitrigel membrane on the side of the culture space 11.

Using this perfusion device 1, the amount of a medium introduced into the perfusion flow path 26 was adjusted by the adjustment part 110 to perform static culture, perfusion culture at a slow flow rate (11 mL/h), and perfusion culture at a fast flow rate (110 mL/h). Thereafter, expression of each gene was examined by real-time PCR.

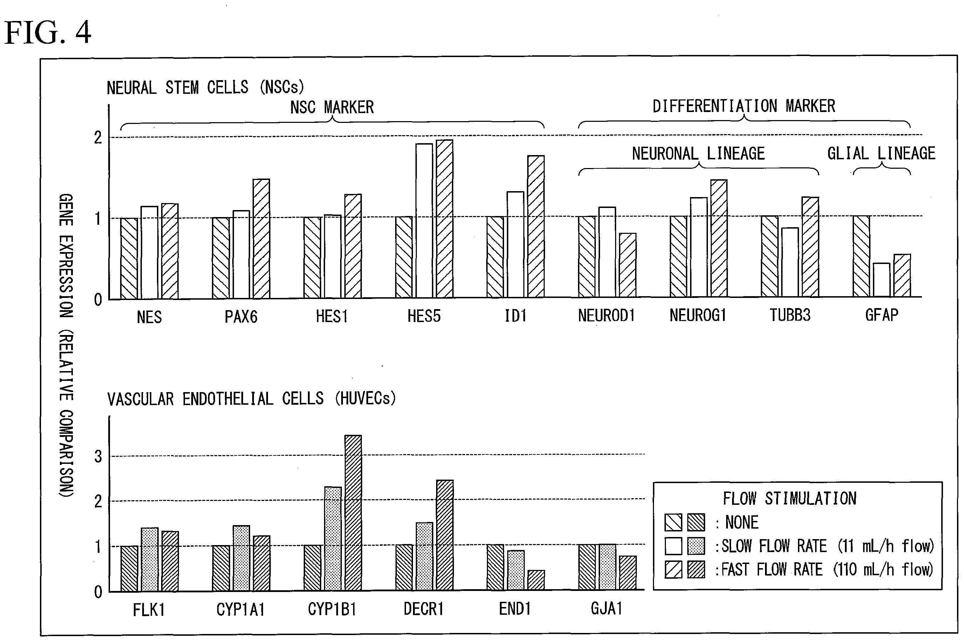

Fig. 4 shows graphs showing the relationship between flow stimulation and gene expression of the neural stem cells (upper part) and the vascular endothelial cells (lower part).

As shown in the lower part of Fig. 4, an increase in gene expression in a flow stimulus-dependent manner was confirmed for CYP1B1 and DECR1, which have been reported to show an increase in gene expression in a flow stimulus-dependent manner. Meanwhile, as shown in the upper part of Fig. 4, an increase in NSC marker expression in a flow stimulus-dependent manner was confirmed for neural stem cells. Therefore, it was shown that stemness of the neural stem cells was enhanced by the flow-stimulated vascular endothelial cells.

### [Experimental Example 2]

In the perfusion device 1 of the above embodiment, the membrane was produced, in which vascular endothelial cells (HUVEC) were bonded to and cultured on the second surface 32 of the gel membrane 30 on the side of the perfusion flow path 26, and hepatoma cells (HepG2) were bonded to and cultured on the first surface 31 of the gel membrane 30 on the side of the culture space 11. As the gel membrane 30, three kinds of a PET membrane, a collagen-coated PET membrane, and a vitrigel membrane were used.

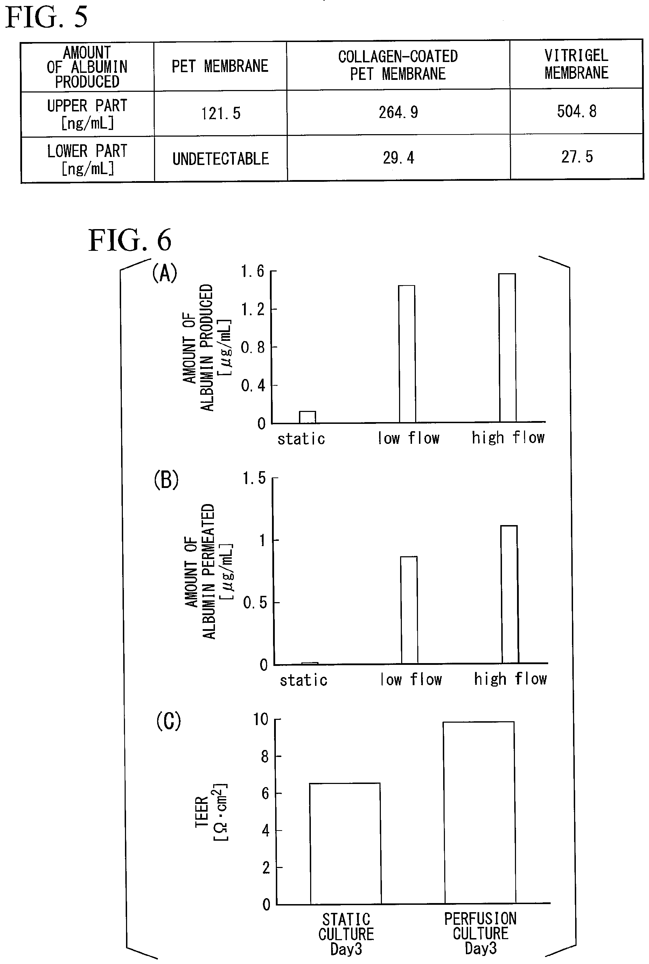

Based on the finding that albumin is released from hepatocytes and released into blood vessels, an amount of albumin produced in a culture space (upper part) and a perfusion flow path (lower part) under perfusion culture was measured for each of the gel membranes 30. Fig. 5 is a table showing measurement results of the amount of albumin produced for each of the gel membranes.

As shown in Fig. 5, it was confirmed that the amount of albumin produced was increased in the culture on the vitrigel membrane. It was also confirmed that albumin permeated the membrane and diffused to the lower part.

Furthermore, in the above perfusion device 1 in which the vitrigel membrane was used, an amount of albumin produced in static culture and perfusion culture, an amount of albumin permeated from the upper part (culture space 11) to the lower part (perfusion flow path 26), and transepithelial electrical resistance (TEER) was measured. As shown in Figs. 6(A) and 6(B), it was confirmed that an amount of albumin produced and an amount of albumin permeated were increased in the perfusion culture. In addition, as shown in Fig. 6(C), it was confirmed that the barrier function was increased by the perfusion.

### [Experimental Example 3]

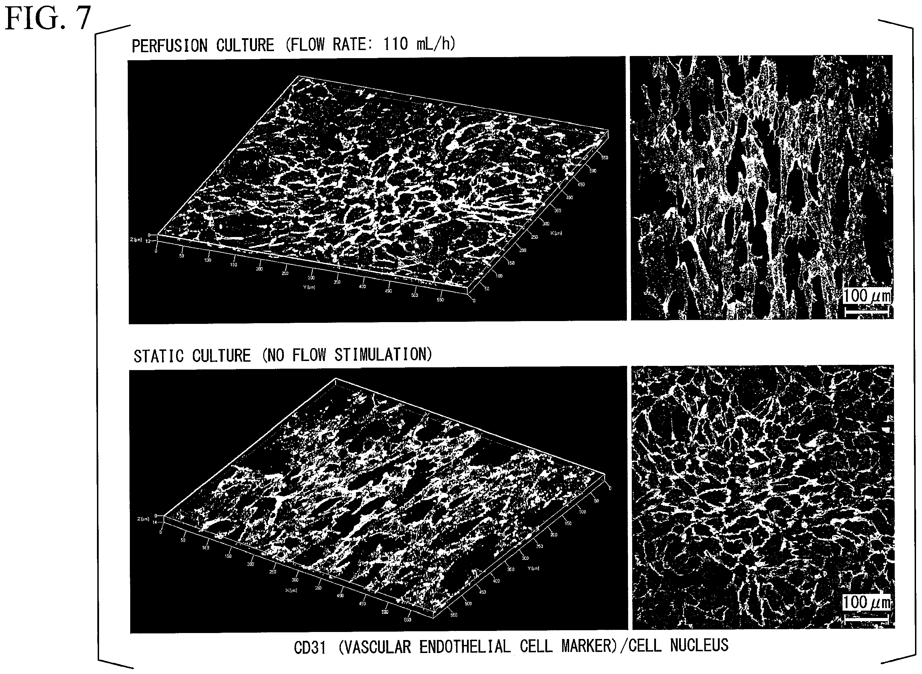

Using the perfusion device 1 produced in Experimental Example 2, cells cultured under static culture and perfusion culture were immunostained with a vascular endothelial marker CD31. Nuclei were stained using DAPI. Fig. 7 is a diagram showing fluorescence images according to flow stimulation of cells cultured under static culture and perfusion culture.

As shown in Fig. 7, orientation of cells by flow stimulation was confirmed under the perfusion culture.

As shown in Experimental Examples 1 to 3, it is possible to easily reproduce environments around the actual luminal layer and to perform comparison, analysis, assessment, and the like relating to co-culture with high accuracy by using the perfusion device 1 in which the perfusion flow path 26 was perfused with a medium but co-culture could be still performed by disposing the luminal cells on the side of the gel membrane 30 facing the perfusion flow path 26 and disposing the tissue-based cells on the side of the gel membrane 30 facing the culture space 11.

Although the preferable embodiments of the present invention have been described above in detail, the present invention is not limited to such specific embodiments, and various modifications and changes are possible within the scope of the gist of the present invention described in the claims.

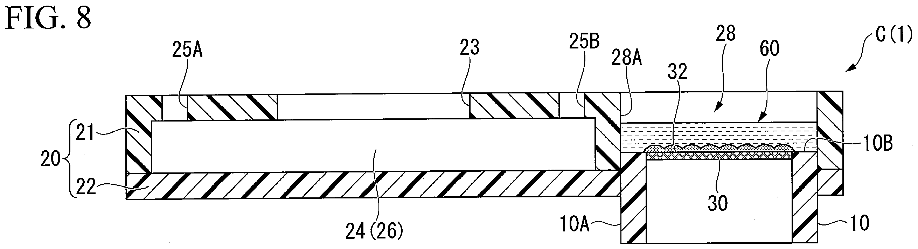

For example, in the above-described embodiment, the configuration in which the cylindrical cap member 40 is used as a pre-culture part has been provided as an exemplary example, but the configuration is not limited to this configuration. As a pre-culture part, for example, it is possible to adopt a configuration in which a through-hole 28, which has an inner peripheral surface 28A fitting with an outer peripheral surface 10A of a well part 10 and which penetrates a base material 20, is provided at a position on the base material 20 which does not interfere with a perfusion flow path 26, as shown in Fig. 8. In the configuration in which the pre-culture part is the through-hole 28, the well part 10 in which a gel membrane 30 faces upward is inserted from a lower opening part of the through-hole 28 and fitted such that a space is formed above the gel membrane 30. Accordingly, a pre-culture space 60 surrounded by the inner peripheral surface 28A, an end surface 10B of the well part 10, and a second surface 32 of the gel membrane 30 is formed.

Unlike the case in which the cap member 40 is used, it is not necessary to distribute and store the base material 20 and the cap member 40 separately in the perfusion device 1 including the through-hole 28 as a pre-culture part. Therefore, it is possible to easily store, distribute, and manage the perfusion device 1.

In addition, in the above-described embodiment, the configuration in which the well part 10 has a cylindrical shape has been provided as an exemplary example, but the configuration is not limited to this configuration as long as the well part 10 has a tubular shape, and the well part 10 may have a square tubular shape with polygonal cross section or a tubular shape with oval cross section. Furthermore, as the well part 10, for example, it is possible to adopt a configuration in which a flange part protruding outward in a radial direction from the outer peripheral surface 10A is provided. As a position of the flange part in a vertical direction, for example, in a case where the well part 10 is held by the holding part 23 of the first base material 21, a position of the second surface 32 of the gel membrane 30 (that is, a position of luminal cells with respect to the perfusion flow path 26) is defined by the flange part engaging with the upper surface of the first base material 21, and in a case where the well part 10 is fitted into and held by the through-hole 28 shown in Fig. 8, a position of the second surface 32 of the gel membrane 30 (that is, a position of luminal cells in the pre-culture space 60) is defined by the flange part engaging with the lower surface of the second base material 22. In this case, because a relative positional relationship of luminal cells to the perfusion flow path 26 and the pre-culture space 60 can be easily maintained, it is possible to minimize fluctuation in a case where a co-culture experiment is performed a plurality of times, and thereby it is possible to perform comparison, analysis, assessment, and the like with high accuracy.

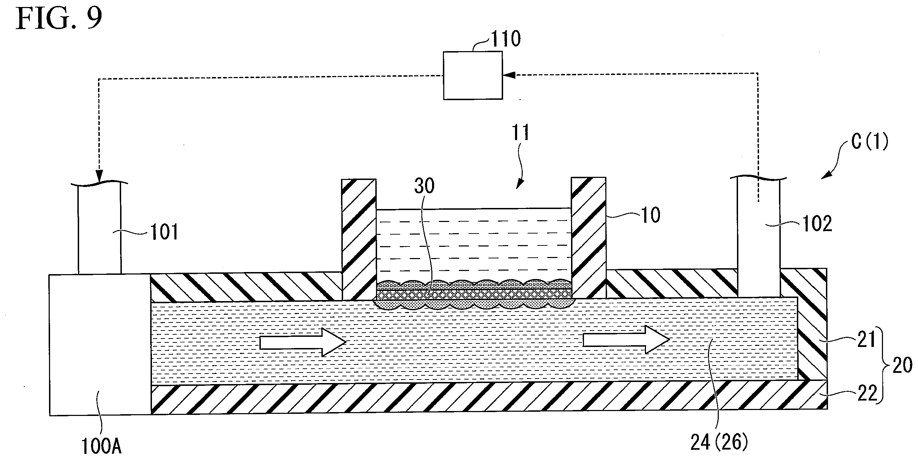

In the above-described embodiment, the configuration in which the medium supply part 100 as a medium reservoir part was provided outside the perfusion device 1 has been provided as an exemplary example, but the configuration is not limited to this configuration. As a medium reservoir part for storing a medium, for example, it is possible to adopt a configuration in which a medium reservoir part 100A, at least a part of which communicates with the upstream side of a perfusion flow path 26, is provided integrally with a base material 20, as shown in Fig. 9. In the case of adopting this configuration, a supply pipe 101 is not connected to the base material 20 but is connected to the medium reservoir part 100A. A medium stored in the medium reservoir part 100A is introduced into the perfusion flow path 26 by driving an adjustment part 110. Then, the medium that perfused the perfusion flow path 26 returns from a discharge pipe 102 to the adjustment part 110 and is supplied again to the medium reservoir part 100A via the supply pipe 101.

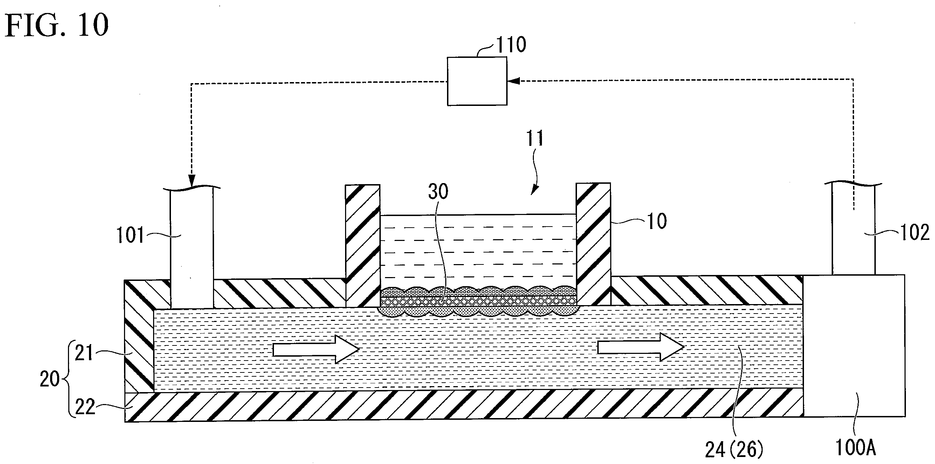

In addition, as the configuration in which the medium reservoir part 100A is integrally provided with the base material 20, it is possible to adopt a configuration in which a medium reservoir part 100A, at least a part of which communicates with the downstream side of a perfusion flow path 26, is provided integrally with a base material 20, as shown in Fig. 10. In the case of adopting this configuration, a supply pipe 101 is connected to the base material 20, and a discharge pipe 102 is not connected to the base material 20 but is connected to the medium reservoir part 100A. A medium stored in the medium reservoir part 100A returns to an adjustment part 110 via the discharge pipe 102 and is introduced into the perfusion flow path 26 via the supply pipe 101 by driving an adjustment part 110. Then, the medium that perfused the perfusion flow path 26 is stored again in the medium reservoir part 100A.

As described above, usability of the perfusion device 1 is improved by providing the medium reservoir part 100A integrally with the base material 20.

In addition, it is possible to adopt a configuration in which a plurality of well parts 10 are provided in series with respect to one perfusion flow path 26. In this case, it is also possible to culture a tissue layer in the plurality of well parts 10 according to a flow direction of a liquid flowing through a luminal layer in a living body. For example, hepatoma cells described in Experimental Example 2 may be cultured in the culture space of the well part 10 disposed on the upstream side of the perfusion flow path 26, and pancreatic tissue may be cultured in the culture space of the well part 10 disposed on the downstream side of the perfusion flow path 26. In this case, it is possible to perform analysis/assessment of an effect of albumin, which has been produced in the upstream well part 10 and then permeated through the membrane and diffused in the perfusion flow path 26, on the pancreatic tissue cultured in the downstream well part 10.

### [Industrial Applicability]

The present invention can be applied to an artificial tissue perfusion device and a method of drug assessment using the artificial tissue perfusion device.

### [Reference Signs List]

- 1: Perfusion device (artificial tissue perfusion device)
- 10: Well part
- 10A: Outer peripheral surface
- 11: Culture space
- 20: Base material
- 23: Holding part
- 26: Perfusion flow path
- 28: Through-hole (pre-culture part)
- 30: Gel membrane
- 31: First surface
- 32: Second surface
- 40: Cap member (pre-culture part)
- 40A: Inner peripheral surface
- 41: Tissue layer
- 42: Luminal layer
- 60: Pre-culture space
- 110: Adjustment part
- C: Co-culture system
- SYS: Perfusion system

## Claims

1. An artificial tissue perfusion device comprising:
a co-culture system in which a plurality of types of cell are cultured,
wherein the co-culture system includes
a tubular well part having a culture space inside,
a base material having a perfusion flow path which extends in a predetermined direction and is perfused with a medium, and a holding part which opens to the perfusion flow path and holds the well part attachably and detachably, and
a gel membrane having a form of a porous membrane and disposed at an end portion of the well part facing the perfusion flow path in a case where the well part is held by the holding part, and
wherein a tissue-based cell is cultured on a first surface side of the gel membrane facing the culture space, and a luminal cell is cultured on a second surface side of the gel membrane facing the perfusion flow path.

2. The artificial tissue perfusion device according to claim 1, wherein the gel membrane contains an extracellular matrix component.

3. The artificial tissue perfusion device according to claim 1 or 2, wherein the luminal cell includes an endothelial cell.

4. The artificial tissue perfusion device according to claim 3, wherein the luminal cell includes a vascular endothelial cell.

5. The artificial tissue perfusion device according to any one of claims 1 to 4, wherein the tissue-based cell includes a neural stem cell.

6. The artificial tissue perfusion device according to any one of claims 1 to 5, wherein a plurality of different types of tissue layer are laminated on the second surface side of the gel membrane.

7. The artificial tissue perfusion device according to any one of claims 1 to 6, further comprising a pre-culture part that has an inner peripheral surface which is freely detachably fitted to an outer peripheral surface from the gel membrane side in the well part separated from the holding part, and that forms a pre-culture space surrounded by the inner peripheral surface fitted to the outer peripheral surface of the well part and by the second surface of the gel membrane.

8. The artificial tissue perfusion device according to claim 7, wherein the pre-culture part has a tubular cap member having the inner peripheral surface.

9. The artificial tissue perfusion device according to claim 7, wherein the pre-culture part has a through-hole that is disposed at a position spaced from the perfusion flow path in the base material, penetrates the base material, and is surrounded by the inner peripheral surface.

10. The artificial tissue perfusion device according to any one of claims 1 to 9, further comprising an adjustment part that is configured to adjust a flow rate of the medium in the perfusion flow path depending on flow stimulation characteristics of at least one of the tissue-based cell and the luminal cell.

11. The artificial tissue perfusion device according to any one of claims 1 to 10, wherein the base material is provided with a medium reservoir part which is configured to store a medium supplied to the perfusion flow path and at least a part of which communicates with the perfusion flow path.

12. A method of drug assessment, the method comprising:
manufacturing an artificial tissue using the artificial tissue perfusion device according to any one of claims 1 to 11;
bringing a drug into contact with the artificial tissue; and
measuring a response of the artificial tissue to a stimulus caused by the contact with the drug or permeability of the drug into the artificial tissue.
